(19) Europäisches Patentamt
European Patent Office
Office européen des brevets

(11) **EP 3 651 820 B1**

(12) **EUROPEAN PATENT SPECIFICATION**

(45) Date of publication and mention
of the grant of the patent:
**25.08.2021 Bulletin 2021/34**

(21) Application number: **18749874.6**

(22) Date of filing: **13.07.2018**

(51) Int Cl.:
*A61M 1/00* (2006.01)          *A61M 3/02* (2006.01)
*A61F 9/007* (2006.01)

(86) International application number:
**PCT/IB2018/055195**

(87) International publication number:
**WO 2019/012494 (17.01.2019 Gazette 2019/03)**

(54) **INFUSION PRESSURE CONTROL SYSTEM**

SYSTEM ZUR INFUSIONSDRUCKSTEUERUNG

SYSTÈME DE CONTRÔLE DE PRESSION DE PERFUSION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.07.2017 IT 201700079495**

(43) Date of publication of application:
**20.05.2020 Bulletin 2020/21**

(73) Proprietor: **Optikon 2000 S.p.A.
00138 Roma (IT)**

(72) Inventors:
• **ROSSI, Tommaso
I-00138 Roma (IT)**
• **QUERZOLI, Giorgio
I-00138 Roma (IT)**
• **ANGELINI, Giovanni Battista
I-00138 Roma (IT)**
• **MALVASI, Carlo
I-00138 Roma (IT)**

(74) Representative: **Chimini, Francesco
Jacobacci & Partners S.p.A.
Piazza della Vittoria 11
25122 Brescia (IT)**

(56) References cited:
**US-A9- 2016 243 304**

EP 3 651 820 B1

## Description

The present invention relates to a system and a method of controlling the ocular infusion pressure for ophthalmic surgery.

As is known, during ophthalmic surgery, an infusion fluid is used for irrigation of the ocular globe. Relevant prior art is for instance disclosed in document US2016/243304.

The present invention relates to a system that, by evaluating the average ocular perfusion pressure measured directly or indirectly during ophthalmic surgery as a function of systemic arterial pressure and intraocular pressure, allows intervening on the infusion pressure in order to prevent blocking the retinal microcirculation and consequent damage and complications to the patient's visual apparatus.

As is known, systemic blood pressure, also called blood pressure (BP), can be distinguished into diastolic blood pressure (DBP) and systolic arterial pressure (SBP) .

Intraocular pressure (IOP) is detectable over time through a measurement system connected to the patient's eye or, during ophthalmic surgery, estimated on the basis of the infusion pressure.

The mean ocular perfusion pressure (MOPP) is calculated over time by combining the values of the aforementioned pressures.

The infusion pressure (IP) is set by the surgeon during the procedure.

By determining the patient's MOPP during the procedure, it is possible to implement a simultaneous regulation of the infusion pressure, and therefore of the intraocular pressure, in order to maintain safety levels that ensure the indemnity of the visual apparatus functions.

It is also possible to visually show the surgeon the range of safe infusion pressure values and emit an alarm if the pressure is outside that range for an excessively long time, before damage to the visual function is caused.

Currently, blood pressure is measured by a pressure gauge connected to a patient's arm. More specifically, during surgery, the values of diastolic blood pressure (DBP) and systolic blood pressure (SBP) of the patient are detected and acquired with a certain frequency. However, this survey has as its sole objective the monitoring of systemic blood pressure for the purposes of surgical practice.

In a mode known as "drop infusion", the infusion pressure is currently set by the surgeon acting on the height of a reservoir containing the infusion solution; so-called "forced infusion" systems are also known which allow setting the desired infusion pressure value.

The current state of the art of ophthalmic surgery equipment is to emit an alarm signal if the infusion pressure is set by the surgeon and maintained for more than one minute, or, in any case, for more than a predetermined time, above a threshold value determined on the basis of statistical considerations and equal for all patients.

However, this type of control, not taking into account the actual systemic arterial pressure of the patient during surgery and more particularly the MOPP, does not exclude the possibility that the infusion pressure reaches levels that slow or stop the retinal microcirculation, causing even permanent damage to the visual system.

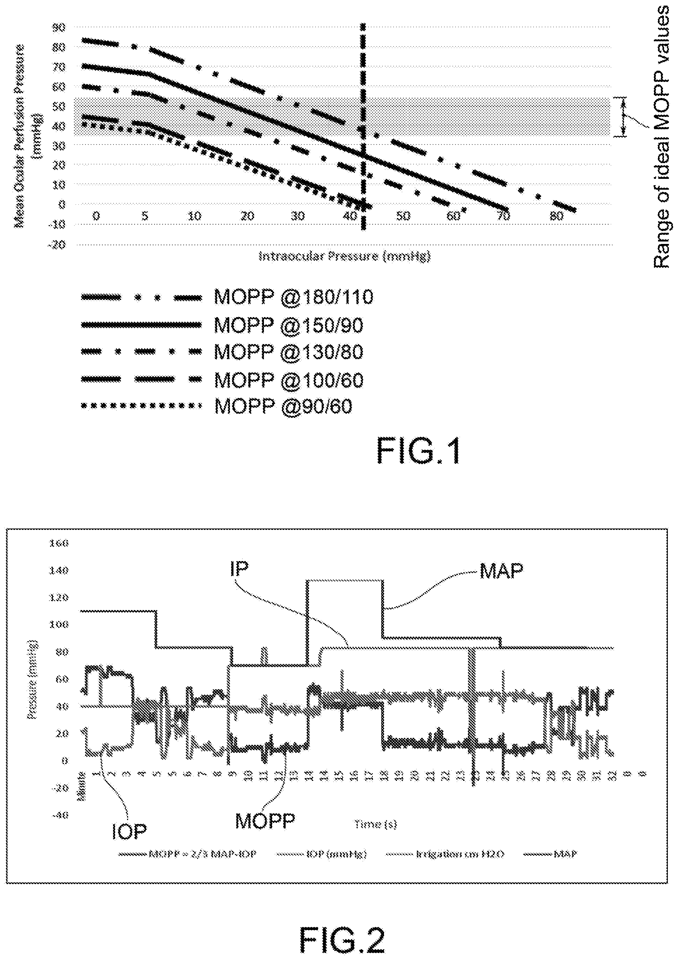

In this regard, the graph in Figure 1 shows some MOPP curves as a function of the IOP relative to subjects with different blood pressure values.

The gray band indicates the zone of ideal MOPP values; if the actual MOPP is below that band, the blood microcirculation in the retina is reduced and, if the condition continues over time, the visual function may be permanently damaged.

It is clear that, for a given value of the IOP (in the graph equal to about 42 mmHg), the MOPP stands outside the range of ideal values identified by the gray band (35÷55 mmHg). The only exception, albeit to the limit, is the MOPP curve at the blood pressure of 180/100.

In almost all cases, conditions of possible suffering on retinal microcirculation are delineated with a possible impairment of the visual apparatus functionality.

It is also clear that even lower IOPs, such as 30mmHg, are "safe" for many patients but can cause damage by reducing retinal microcirculation in patients with insufficiently high MOPP values.

The graph in Figure 2 shows, on the other hand, that IOP values above 35 mmHg are common during a surgical session. In particular, the trends of MOPP, IOP, IP and MAP are reported as a function of time during a combined phacoemulsification, vitrectomy (PPV) and pucker surgery with final air/balanced saline exchange.

It can be seen that, for more than half the duration of the intervention (18 minutes out of 32), the MOPP is below the IOP and 30 mmHg, well below the ideal minimum limit.

From the above considerations, it is clear that the system adopted by current ophthalmic surgery machines to set an alarm threshold for the maximum IP based on statistical considerations, equal for all patients, involves a residual risk of damage to vision for the patient range that have a lower blood pressure, and therefore lower MOPP.

The object of the present invention is therefore to provide a system capable of carrying out a control of the infusion pressure, therefore, of the IOP, specific for the individual patient, in order to prevent blockage of the retinal circulation and the consequent damage to the patient's visual apparatus.

Such an object is achieved by an infusion pressure control system according to claim 1.

The dependent claims describe preferred embodiments of the invention.

According to claim 1, the infusion pressure control system comprises intraocular pressure detection means, arterial pressure detection means and a control unit operatively connected to said intraocular pressure detection means and to said blood pressure detection means.

The control unit is configured for calculating the mean ocular perfusion pressure value based on the intraocular pressure and arterial pressure values provided by the intraocular pressure detection means and by the arterial pressure detection means, and for comparing the calculated mean ocular perfusion pressure value and at least one predetermined threshold value.

In one embodiment, the control unit is configured to generate an alarm signal if the calculated mean ocular perfusion pressure value is below the predetermined threshold value.

According to an aspect of the invention, the control system comprises an electromedical apparatus adapted to be connected to the patient at least by means of an infusion line suitable for dispensing an infusion fluid at an infusion pressure. The electromedical apparatus is suitable to allow an adjustment of the infusion pressure.

In one embodiment, the control unit is integrated into the electromedical apparatus.

For example, the electromedical apparatus is a phacoemulsifier or a phacovitrectomer.

In one embodiment, the intraocular pressure detection means comprise an intraocular pressure sensor.

In one embodiment, the intraocular pressure detection means are suitable for calculating the intraocular pressure value based on the infusion pressure value of an infusion fluid.

In one embodiment, the infusion line comprises a gravitational or forced infusion system by means of pressurizing a vessel containing the infusion fluid.

In one embodiment, the means for detecting arterial pressure include a pressure gauge suitable to be connected to the patient.

In one embodiment, the means of detecting arterial pressure are connected to the control unit via a wireless communication system.

In one embodiment, the control unit is configured to command the infusion line to reduce the infusion pressure if the value of the calculated mean ocular perfusion pressure is below the predetermined threshold value.

In one embodiment, the electromedical apparatus is provided with an operator interface suitable to allow a manual adjustment of the infusion pressure.

In one embodiment, the mean ocular perfusion pressure value is calculated continuously or at predetermined time intervals.

In one embodiment, the mean ocular perfusion pressure (MOPP) value is calculated using the formula:

$$MOPP = 115/130 \; MAP-IOP,$$

or using the formula

$$MOPP = 2/3 \; MAP-IOP,$$

where MAP indicates mean arterial pressure, for example calculated according to the formula MAP = 1/3 SBP + 2/3 DBP, and IOP means the intraocular pressure.

The IOP, during ocular surgery, is determined by the pressure of the saline solution introduced into the eye continuously during the operation.

According to the invention, the control unit comprises a timer operatively connected to an alarm. The timer is incremented and decremented on the basis of a comparison between the value of the average ocular perfusion pressure calculated and the at least one predetermined threshold value. The alarm is activated when the timer exceeds a pre-set timer threshold value.

In one embodiment, the control unit comprises or is operatively linked to a graphic interface suitable for displaying an alarm message or an indicator capable of generating an audible signal if the average pressure value of the calculated mean ocular perfusion pressure is lower than the predetermined threshold value.

According to the invention, the control unit comprises or is operatively connected to a user interface suitable for receiving a consent command from the operator, said consent command being suitable to enable the infusion pressure adjustment.

In one embodiment, the infusion pressure is adjustable by varying the height of the reservoir containing the infusion fluid.

In one embodiment, the infusion pressure is adjustable by varying a controlled overpressure in use upstream of the infusion fluid.

A method of regulating the infusion pressure is also an object of the present disclosure, comprising the steps of:

- detecting the intraocular pressure;
- detecting the blood pressure;
- calculating the mean ocular perfusion pressure value on the basis of the intraocular pressure and blood pressure values measured; and
- carrying out a comparison between said value of the average ocular perfusion pressure calculated and at least one predetermined threshold value.

According to an aspect, the control method provides for generating an alarm signal if the calculated mean ocular perfusion pressure value is below the predetermined threshold value.

In one embodiment, the control method provides for commanding the infusion line of an electromedical equipment to reduce the infusion pressure if the value of the calculated mean ocular perfusion pressure is below the predetermined threshold value.

According to an aspect, the control method provides for increasing or decreasing a timer operatively connected to an alarm, based on the comparison between the mean ocular perfusion pressure value calculated and a predetermined threshold value, and activating the alarm when the timer exceeds a pre-set timer threshold value.

In one embodiment, the control method provides for displaying an alarm message on a graphical interface or generating an audible signal by means of a sound signaler if the mean ocular perfusion pressure value calculated is lower than the pre-set threshold value.

A specific object of the present disclosure therefore is a system for ophthalmic surgery, comprising an electromedical apparatus connected to the patient's eye at least by a fluid infusion line, preferably capable of adjusting the infusion pressure of said fluids and/or establishing an alarm threshold for said pressure based on the blood pressure value detected on the patient by means of suitable measuring means and the detected or calculated IOP value, so as to keep the MOPP above the safety threshold.

In its preferred implementation, the invention is implemented with an electromedical apparatus for phacoemulsification and/or for vitrectomy.

This apparatus is preferably provided with control software and/or firmware and a user interface that includes an alarm system.

Furthermore, said apparatus may comprise a system capable of varying the pressure of the fluid used for infusion into the ocular globe by varying the height of the reservoir containing the fluid itself and/or pressurizing the reservoir.

The irrigation fluid may be a liquid, for example a saline solution, or a gas, for example sterile air.

The apparatus object of the present disclosure is, moreover, connected to a device capable of detecting the systolic blood pressure (SBP) and the diastolic blood pressure (DBP) of the patient; said connection may be wired or wireless.

The apparatus may be provided with a patient's ocular pressure measurement system (IOP) or calculate this pressure according to the infusion pressure, the impedance of the connecting pipes and the irrigated flow.

The features and the advantages of the infusion pressure control system according to the invention and of the method according to the present disclosure shall be made readily apparent from the following description of preferred embodiments thereof, provided purely by way of a non-limiting example, with reference to the accompanying figures, in which:

- Figure 1 is a graph showing some MOPP curves as a function of the IOP relative to subjects with different blood pressure values;
- Figure 2 is a graph showing the trend of some characteristic pressures during an ophthalmic surgery;
- Figure 3 is a schematic representation of the infusion pressure control system according to the invention;

- Figure 4 is a flowchart of an example of infusion pressure regulation logic according to the present invention; and
- Figure 5 is an example of a graphical user interface for the representation and regulation of the infusion pressure.

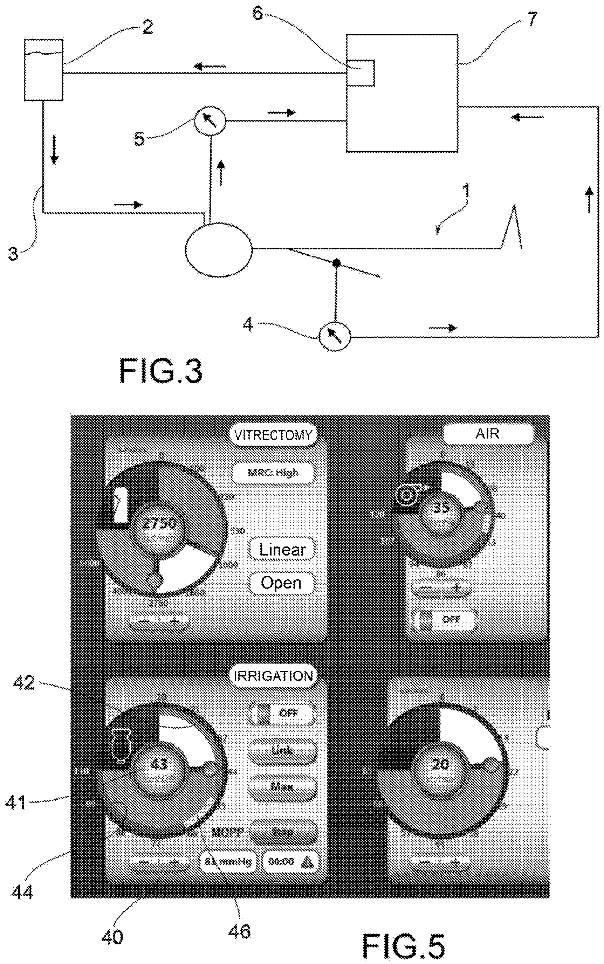

With reference to Figure 3, reference numeral 1 diagrammatically shows a patient to whom a blood pressure meter 4 is connected.

The blood pressure data is sent to an electromedical apparatus 7.

An intra-ocular pressure meter 5 allows direct detection of the intraocular pressure (IOP). The intraocular pressure data is transmitted to the electromedical apparatus 7.

If the conditions foreseen by the regulation logic are met, the control unit of the electromedical apparatus 7, for example after receiving the authorization by the surgeon, commands pressure control means 6. These pressure control means 6 intervene on the infusion line 3, varying the pressure of the infusion fluid contained in the reservoir 2, until the value of the MOPP is brought back within the safety limits.

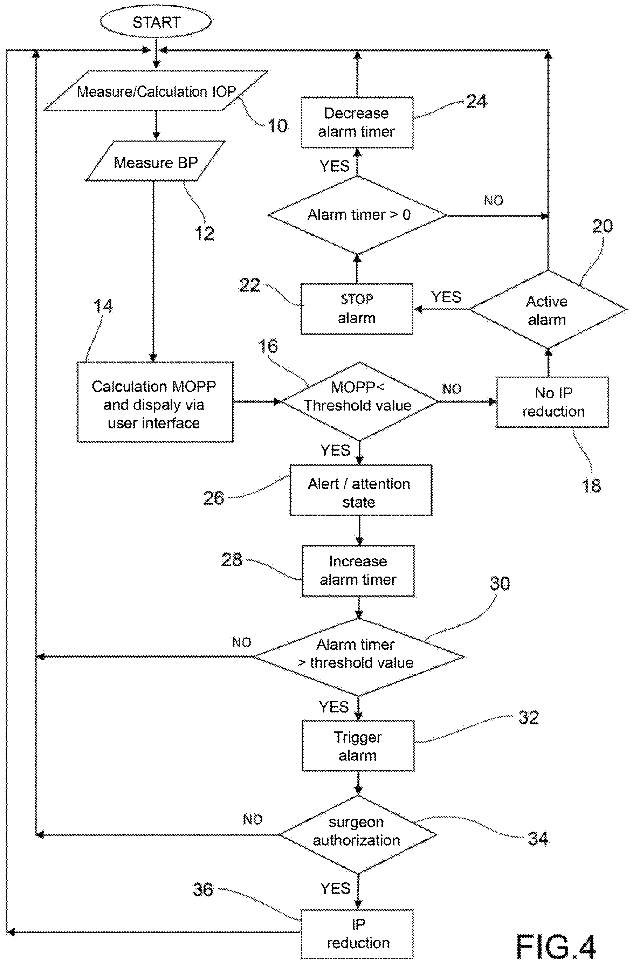

Figure 4 describes the flow chart of the infusion pressure regulation method.

Initially, the intraocular pressure (IOP) is measured (step 10). This can be determined once the position of the reservoir 2 with the irrigation fluid is known or can be measured with a pressure sensor connected to the patient's eye.

Subsequently, blood pressure (BP) is measured (step 12) .

By combining the IOP and BP pressure values, the system calculates the MOPP and provides it to the surgeon via the user interface (step 14). The MOPP is then compared to a threshold value (step 16).

If the MOPP is greater than the threshold value, the infusion pressure is not reduced (step 18).

The status of the alarm is then checked (step 20) . If the system alarm is not active, the cycle returns to the beginning.

If the system is in alarm, the alarm is stopped (step 22). The cycle starts again after decreasing the alarm timer, if this was greater than zero (step 24).

If the MOPP is lower than the threshold value, the system goes into an alert state (step 26) and the alarm timer is increased (step 28).

The alarm timer is then compared to a timer threshold value (step 30).

If the alarm timer is greater than the threshold value, the system triggers an alarm (step 32), otherwise it returns to the beginning of the cycle.

If the alarm is active, the consequent adjustment of the infusion pressure (step 36) may be subject to the authorization of the surgeon which is carried out through the user interface of the electromedical apparatus (step 34) .

Figure 5 shows an example of a user interface suitable for showing the infusion pressure to the surgeon for maintaining the ideal ocular pressure for a correct Mean Ocular Perfusion Pressure (MOPP) and allowing pressure regulation.

The button for setting the infusion pressure is indicated with reference numeral 40; the indicator of the current infusion pressure value is indicated with reference numeral 41. Zone 42, for example colored green, around indicator 41 indicates the range of infusion pressures that cause an optimal MOPP; zone 44, for example colored red, indicates the zone of infusion pressure which generates a definitely insufficient MOPP; zone 46, for example colored yellow, indicates an alarm zone but not of immediate danger.

**Claims**

1. An infusion pressure control system for ophthalmic surgery, including:

   - intraocular pressure detection means (5);
   - arterial pressure detection means (4); and
   - a control unit operatively connected to said intraocular pressure detection means and said arterial pressure detection means and configured for calculating the mean ocular perfusion pressure value based on the intraocular pressure and arterial pressure values provided by said intraocular pressure detection means and by said arterial pressure detection means, and for comparing said calculated mean ocular perfusion pressure value and at least one predetermined threshold value,

   **characterized in that** the control unit comprises a timer operatively linked to an alarm, the timer being incrementable and decrementable based on comparing the value of the calculated mean ocular perfusion pressure and the at least one predetermined threshold value, the alarm being activated when the timer exceeds a predetermined timer threshold value,
   and wherein the control unit comprises or is operatively connected to a user interface suitable for receiving a consent command from the operator, said consent command being suitable to enable an infusion pressure adjustment.

2. A system according to claim 1, wherein the control unit is configured to generate an alarm signal if the calculated mean ocular perfusion pressure value is below the predetermined threshold value.

3. A system according to claim 1 or 2, comprising an electromedical apparatus (7) adapted to be connected to the patient (1) at least by means of an infusion line (3) suitable for dispensing an infusion fluid at an infusion pressure, said electromedical apparatus being adapted to allow adjustment of said infusion pressure.

4. A system according to any one of the preceding claims, wherein said intraocular pressure detection means comprise an intraocular pressure sensor.

5. A system according to any one of the preceding claims, wherein said intraocular pressure detection means are suitable for calculating the intraocular pressure value based on the pressure value and, optionally, the infusion flow of an infusion fluid.

6. A system according to any one of the claims 3-5, wherein the electromedical apparatus is a phacoemulsifier or a phacovitrectomer.

7. A system according to any one of claims 3-6, wherein the infusion line comprises a gravitational or forced infusion system by means of pressurizing a vessel containing the infusion fluid.

8. A system according to any one of the preceding claims, wherein the means for detecting arterial pressure include a pressure gauge suitable to be connected to the patient.

9. A system according to any one of the preceding claims, wherein the means of detecting arterial pressure are connected to the control unit via a wireless communication system.

10. A system according to any one of claims 3-9, wherein the control unit is configured to command the infusion line to reduce the infusion pressure if the value of the calculated mean ocular perfusion pressure is below the predetermined threshold value.

11. A system according to any one of claims 3-9, wherein the electromedical apparatus is provided with an operator interface suitable to allow a manual adjustment of the infusion pressure.

12. A system according to any one of the preceding claims, wherein the mean ocular perfusion pressure value is calculated continuously or at predetermined time intervals.

13. A system according to any one of the preceding claims, wherein the mean ocular perfusion pressure (MOPP) value is calculated using the formula:

$$MOPP = 115/130\ MAP-IOP,$$

or using the formula:

$$MOPP = 2/3 \; MAP-IOP,$$

where MAP indicates mean arterial pressure, for example calculated according to the formula MAP = 1/3 SBP + 2/3 DBP, and IOP means the intraocular pressure.

14. A system according to any one of the preceding claims, wherein the control unit comprises or is operatively linked to a graphic interface suitable for displaying an alarm message or an indicator capable of generating an audible signal if the average pressure value of the calculated mean ocular perfusion pressure is lower than the predetermined threshold value.

15. A system according to any one of claims 3-14, wherein the infusion pressure is adjustable by varying the height of the reservoir (2) containing the infusion fluid.

16. A system according to any one of claims 3-14, wherein the infusion pressure is adjustable by varying a controlled overpressure in use upstream of the infusion fluid.

**Patentansprüche**

1. Infusionsdrucksteuerungs- bzw. -regelungssystem für Augenchirurgie, enthaltend:

    - Augeninnendruckerfassungsmittel (5);
    - Arteriendruckerfassungsmittel (4); und
    - eine Steuerungs- bzw. Regelungseinheit, die operativ mit den Augeninnendruckerfassungsmitteln und den Arteriendruckerfassungsmitteln verbunden ist und konfiguriert ist zum Berechnen des mittleren Augenperfusionsdruckwertes basierend auf den Augeninnendruck- und Arteriendruckwerten, die von den Augeninnendruckerfassungsmitteln und den Arteriendruckerfassungsmitteln bereitgestellt werden, und zum Vergleichen des berechneten mittleren Augenperfusionsdruckwertes und zumindest eines vorbestimmten Schwellenwerts, **dadurch gekennzeichnet, dass** die Steuerungs- bzw. Regelungseinheit einen Timer umfasst, der operativ mit einem Alarm verbunden ist, wobei der Timer basierend auf dem Vergleichen des Wertes des berechneten mittleren Augenperfusionsdrucks und des zumindest einen vorbestimmten Schwellenwerts inkrementierbar und dekrementierbar ist, wobei der Alarm aktiviert wird, wenn der Timer einen vorgegebenen Timer-Schwellenwert überschreitet,

und wobei die Steuerungs- bzw. Regelungseinheit eine Benutzerschnittstelle umfasst oder mit dieser operativ verbunden ist, die geeignet ist, einen Zustimmungsbefehl von der Bedienungsperson zu empfangen, wobei der Zustimmungsbefehl geeignet ist, eine Infusionsdruckeinstellung zu ermöglichen.

2. System nach Anspruch 1, wobei die Steuerungs- bzw. Regelungseinheit konfiguriert ist, ein Alarmsignal zu erzeugen, wenn der berechnete mittlere Augenperfusionsdruckwert unter dem vorbestimmten Schwellenwert liegt.

3. System nach Anspruch 1 oder 2, umfassend eine elektromedizinische Vorrichtung (7), die angepasst ist, mit dem Patienten (1) zumindest mittels einer Infusionsleitung (3) verbunden zu werden, die zum Abgeben einer Infusionsflüssigkeit mit einem Infusionsdruck geeignet ist, wobei die elektromedizinische Vorrichtung angepasst ist, eine Einstellung des Infusionsdrucks zu erlauben.

4. System nach einem der vorhergehenden Ansprüche, wobei die Augeninnendruckerfassungsmittel einen Augeninnendrucksensor umfassen.

5. System nach einem der vorhergehenden Ansprüche, wobei die Augeninnendruckerfassungsmittel geeignet sind, den Augeninnendruckwert basierend auf dem Druckwert und optional dem Infusionsfluss einer Infusionsflüssigkeit zu berechnen.

6. System nach einem der Ansprüche 3-5, wobei die elektromedizinische Vorrichtung ein Phakoemulgator oder ein Phakovitrektomer ist.

7. System nach einem der Ansprüche 3-6, wobei die Infusionsleitung ein Gravitations- oder Zwangsinfusionssystem mittels Druckbeaufschlagung eines die Infusionsflüssigkeit enthaltenden Gefäßes umfasst.

8. System nach einem der vorhergehenden Ansprüche, wobei die Mittel zum Erfassen des arteriellen Drucks einen Druckmesser enthalten, der geeignet ist, mit dem Patienten verbunden zu werden.

9. System nach einem der vorhergehenden Ansprüche, wobei die Mittel zum Erfassen des arteriellen Drucks über ein drahtloses Kommunikationssystem mit der Steuerungs- bzw. Regelungseinheit verbunden sind.

10. System nach einem der Ansprüche 3-9, wobei die Steuerungs- bzw. Regelungseinheit konfiguriert ist, der Infusionsleitung zu befehlen, den Infusionsdruck zu reduzieren, falls der Wert des berechneten mittleren Augenperfusionsdrucks unter dem vorbestimmten Schwellenwert liegt.

**11.** System nach einem der Ansprüche 3-9, wobei die elektromedizinische Vorrichtung mit einer Benutzerschnittstelle versehen ist, die geeignet ist, eine manuelle Einstellung des Infusionsdrucks zu erlauben.

**12.** System nach einem der vorhergehenden Ansprüche, wobei der mittlere Augenperfusionsdruckwert kontinuierlich bzw. durchgehend oder in vorbestimmten Zeitintervallen berechnet wird.

**13.** System nach einem der vorhergehenden Ansprüche, wobei der mittlere Augenperfusionsdruck(MOPP)-Wert unter Verwendung der Formel:

$$MOPP = 115/130\ MAP\text{-}IOP$$

oder unter Verwendung der Formel:

$$MOPP = 2/3\ MAP\text{-}IOP$$

berechnet wird,
wobei MAP den mittleren arteriellen Druck angibt, beispielsweise gemäß der Formel MAP = 1/3 SBP + 2/3 DBP berechnet, und IOP den Augeninnendruck bedeutet.

**14.** System nach einem der vorhergehenden Ansprüche, wobei die Steuerungs- bzw. Regelungseinheit eine graphische Schnittstelle, die geeignet ist, eine Alarmmeldung anzuzeigen, oder einen Indikator umfasst oder operativ damit verbunden ist, der in der Lage ist, ein hörbares bzw. akustisches Signal zu erzeugen, falls der Durchschnittsdruckwert des berechneten mittleren Augenperfusionsdrucks niedriger als der vorbestimmte Schwellenwert ist.

**15.** System nach einem der Ansprüche 3-14, wobei der Infusionsdruck durch Variieren der Höhe des Reservoirs (2), welches das Infusionsfluid enthält, einstellbar ist.

**16.** System nach einem der Ansprüche 3-14, wobei der Infusionsdruck durch Variieren eines kontrollierten Überdrucks im Gebrauch stromaufwärts des Infusionsfluids einstellbar ist.

## Revendications

**1.** Système de commande de pression de perfusion pour chirurgie ophtalmique, comprenant :

- des moyens de détection de pression intraoculaire (5) ;
- des moyens de détection de pression artérielle (4) ; et

- une unité de commande fonctionnellement raccordée auxdits moyens de détection de pression intraoculaire et auxdits moyens de détection de pression artérielle et configurée pour calculer la valeur de pression de perfusion oculaire moyenne sur la base des valeurs de pression intraoculaire et de pression artérielle fournies par lesdits moyens de détection de pression intraoculaire et par lesdits moyens de détection de pression artérielle, et pour comparer ladite valeur de pression de perfusion oculaire moyenne calculée et au moins une valeur seuil prédéterminée, **caractérisé en ce que**

l'unité de commande comprend un chronomètre fonctionnellement lié à une alarme, le chronomètre pouvant être incrémenté et décrémenté sur la base d'une comparaison de la valeur de la pression de perfusion oculaire moyenne calculée et de l'au moins une valeur seuil prédéterminée, l'alarme étant activée lorsque le chronomètre excède une valeur seuil de chronomètre prédéterminée,
et dans lequel l'unité de commande comprend ou est fonctionnellement raccordée à une interface utilisateur adaptée à recevoir une commande de consentement de l'opérateur, ladite commande de consentement étant adaptée à permettre un ajustement de la pression de perfusion.

**2.** Système selon la revendication 1, dans lequel l'unité de commande est configurée pour générer un signal d'alarme si la valeur de pression de perfusion oculaire moyenne calculée est inférieure à une valeur seuil prédéterminée.

**3.** Système selon la revendication 1 ou 2, comprenant un appareil électromédical (7) adapté à être raccordé au patient (1) au moins au moyen d'une ligne de perfusion (3) adaptée à administrer un fluide de perfusion à une pression de perfusion, ledit appareil électromédical étant adapté à permettre le réglage de ladite pression de perfusion.

**4.** Système selon l'une quelconque des revendications précédentes, dans lequel lesdits moyens de détection de pression intraoculaire comprennent un capteur de pression intraoculaire.

**5.** Système selon l'une quelconque des revendications précédentes, dans lequel lesdits moyens de détection de pression intraoculaire sont adaptés à calculer la valeur de pression intraoculaire sur la base de la valeur de pression et, facultativement, le débit de perfusion d'un fluide de perfusion.

**6.** Système selon l'une quelconque des revendications 3 à 5, dans lequel l'appareil électromédical est un phacoémulsificateur ou un phacovitréotome.

**7.** Système selon l'une quelconque des revendications 3 à 6, dans lequel la ligne de perfusion comprend un système de perfusion gravitationnelle ou forcée au moyen de la pressurisation d'un récipient contenant le fluide de perfusion.

**8.** Système selon l'une quelconque des revendications précédentes, dans lequel les moyens de détection de pression artérielle incluent une jauge de pression adaptée à être raccordée au patient.

**9.** Système selon l'une quelconque des revendications précédentes, dans lequel les moyens de détection de pression artérielle sont raccordés à l'unité de commande via un système de communication sans fil.

**10.** Système selon l'une quelconque des revendications 3 à 9, dans lequel l'unité de commande est configurée pour commander à la ligne de perfusion de réduire la pression de perfusion si la valeur de la pression de perfusion oculaire moyenne calculée est inférieure à la valeur seuil prédéterminée.

**11.** Système selon l'une quelconque des revendications 3 à 9, dans lequel l'appareil électromédical est doté d'une interface opérateur adaptée à permettre un réglage manuel de la pression de perfusion.

**12.** Système selon l'une quelconque des revendications précédentes, dans lequel la valeur de pression de perfusion oculaire moyenne est calculée en continu ou à des intervalles de temps prédéterminés.

**13.** Système selon l'une quelconque des revendications précédentes, dans lequel la valeur de pression de perfusion oculaire moyenne (MOPP) est calculée en utilisant la formule :

$$MOPP = 115/130 \, MAP - IOP,$$

ou en utilisant la formule :

$$MOPP = 2/3 \, MAP - IOP,$$

où MAP indique une pression artérielle moyenne, par exemple calculée conformément à la formule MAP = 1/3 SBP + 2/3 DBP, et IOP signifie la pression intraoculaire.

**14.** Système selon l'une quelconque des revendications précédentes, dans lequel l'unité de commande comprend ou est fonctionnellement liée à une interface graphique adaptée à afficher un message d'alarme ou un indicateur capable de générer un signal audible si la valeur de pression moyenne de la pression de perfusion oculaire moyenne calculée est inférieure à la valeur seuil prédéterminée.

**15.** Système selon l'une quelconque des revendications 3 à 14, dans lequel la pression de perfusion est ajustée en faisant varier la hauteur du réservoir (2) contenant le fluide de perfusion.

**16.** Système selon l'une quelconque des revendications 3 à 14 précédentes, dans lequel la pression de perfusion peut être ajustée en faisant varier une surpression commandée durant l'utilisation en amont du fluide de perfusion.

FIG.1

FIG.2

FIG.3

FIG.5

FIG.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016243304 A